# EUROPÄISCHE PATENTANMELDUNG

(11) **EP 3 498 306 A1**
(43) Veröffentlichungstag der Anmeldung: **19.06.2019**
(21) Anmeldenummer: 17207880.0
(22) Anmeldetag: 16.12.2017
(51) Int. Cl.: A61K 49/00, A61B 5/00, A61K 36/53

(54) **EXTRAKTE AUS VITEX AGNUS CASTUS ZUR BEHANDLUNG UND DIAGNOSE VON BRUSTKREBS**

(71) Anmelder: Bionorica SE, 92318 Neumarkt (DE)
(72) Erfinder: WUTTKE, Wolfgang, 37120 Bovenden (DE); SEIDLOVÁ-WUTTKE, Dana, 37120 Bovenden (DE)
(74) Vertreter: Simandi, Claus

(57) **Zusammenfassung**

Die vorliegende Erfindung betrifft Dopamin-Agonisten, insbesondere Pflanzenextrakte aus Vitex Agnus Castus, und zwar ein Arzneimittel zur Behandlung von Brustkrebs als auch ein Mittel zur Diagnose von Brustkrebs mithilfe der Mammographie.

## Beschreibung

Die vorliegende Erfindung betrifft Dopamin-Agonisten, insbesondere Pflanzenextrakte aus Vitex Agnus Castus, und zwar ein Arzneimittel zur Behandlung von Brustkrebs als auch ein Mittel zur Diagnose von Brustkrebs mithilfe der Mammographie.

Pflanzenextrakte aus Vitex Agnus Castus (Synonym: Mönchspfeffer, Keuschlamm) sind als Arzneimittel zur Behandlung von Regeltempoanomalien, Mastodynie, Prämenstruellen Syndrom (PMS), Störungen der Regelblutung (infolge primärer und sekundärer Gelbkörperinsuffizienz), insbesondere Dysmenorrhoe beschrieben. Die Anmelderin vertreibt entsprechende Arzneimittel auf Basis eines Pflanzenextrakts aus Vitex Agnus Castus mit den Marken Mastodynon® und Agnucaston®.

Weiterhin werden solche Pflanzenextrakte aus Vitex Agnus Castus zur Östrogenersatztherapie eingesetzt (WO 2003/068199 A1) und wirken analog dem körpereigenen Botenstoff Dopamin und können infolgedessen die Ausschüttung des Hormons Prolaktin senken und dienen daher als Prolaktin-senkendes Mittel.

Ferner beschreibt EP 1 131 061 B1 besonders geeignete bicyklische Diterpene als Prolaktin-senkendes Mittel, die aus dem Pflanzenextrakt Vitex Agnus Castus isoliert werden.

Pflanzenextrakte aus Vitex Agnus Castus sind daher ebenso wie Dopamin in der Lage, Dopamin-Rezeptoren (D-Rezeptoren) zu stimulieren und gelten daher als Dopamin-Agonisten. Dopaminagonisten werden vorzugsweise in der Behandlung von Parkinson und anderen neurodegenerativen Erkrankungen eingesetzt.

Allgemein gelten Dopamin-Agonisten zudem als Prolaktinhemmer (-senker). Es wird unterschieden zwischen ergolinen und nicht-ergolinen Dopamin-Agonisten. Ergoline Dopamin-Agonisten leiten sich als Derivate von Ergolin ab, dessen Struktur das Grundgerüst der Mutterkornalkaloide ist, wie nicht abschließend Bromocriptin und Cabergolin. Nichtergoline Substanzen sind chemisch heterogen und solche wie nicht abschließend Apomorphin, Pramipexol, Quinagolid, Ropinirol oder Rotigotin.

Im Stand der Technik ist beschrieben, dass eine Erhöhung des Prolaktin-Spiegels das Risiko für Brustkrebs erhöht (Tikk et al, Breast Cancer Research 2015, 17:49-57). Frauen mit einer hohen Brustdichte weisen häufig einen erhöhten Prolaktin-Spiegel auf (Rice et al, Breast Cancer Res Treat, 2015 149: 245-253). Weiterhin ist aus der Women's Health Initiative (WHI)-Studie bekannt, dass Probanden in der Östrogen/Gestagen-Gruppe, welche unter der Therapie eine Mastodynie entwickelten, im Verlauf von 7 Jahren signifikant häufiger an Brustkrebs erkrankten als Frauen in der Plazebogruppe (Crandall et al Breast Cancer Res Treat, 2012, 131: 969-979; und 132: 275-286).

Die Brustdichte (mammographische Dichte) wird standardmäßig (ACR-Stufen 1-4) wie folgt eingeteilt:

**Tabelle 1:**

| Stufe | Definition |
|---|---|
| 1 | Die Brust besteht fast vollständig aus Fettgewebe; |
| 2 | Die Brust enthält vereinzelte Bereiche mit dichtem Drüsen- und Bindegewebe; |
| 3 | Die Brust ist unregelmäßig dicht (mehr Drüsen- und Bindegewebe als Fettgewebe); |
| 4 | Die Brust ist extrem dicht und besteht fast ausschließlich aus Drüsen- und Bindegewebe; |

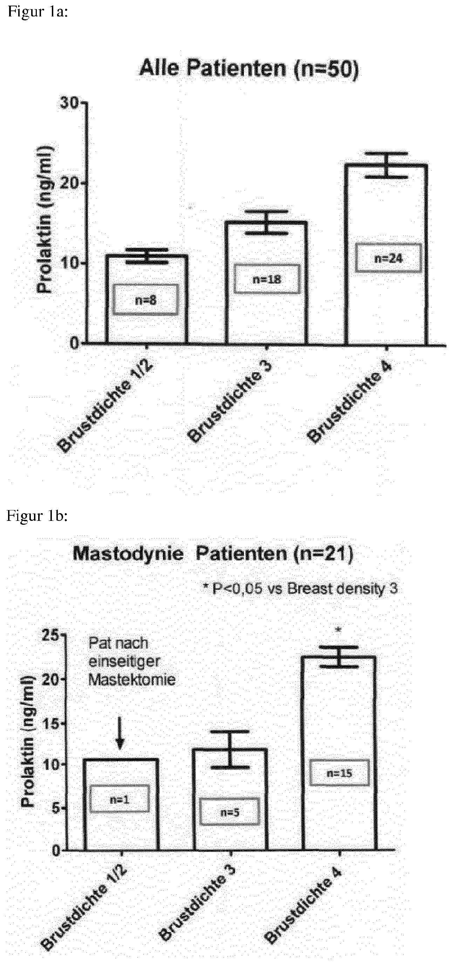

Eine hohe Brustdichte (ACR Stufe 3-4) gilt als Risikofaktor für Brustkrebs (Stone et al, Am. J. Epidemiol. 2009, 170: 1571-1578, Warwick et al, Breast Cancer Research 2014, 16: 451-457). Prolaktin ist mindestens ein verantwortlicher Faktor zur Erhöhung der Brustdichte, da die Teilung des Epithels in der Brustdrüse stimuliert wird, und Lobuli/Lobi im Drüsengewebe angereichert werden. Ein entsprechender Zusammenhang ist in der Figur 1a dargelegt, ebenfalls im Fall einer Mastodynie, siehe Figur 1b.

Es ist jedoch im Stand der Technik problematisch, und bisher nicht erfolgreich gelöst, dass eine hohe Brustdichte eine Mammographie erschwert, insbesondere die Identifizierung und Beurteilung eines Mammakarzinoms, also die Diagnose eines Mammakarzinoms.

Eine hohe Brustdichte in den Stufen 3-4 kann dazu führen, dass im Zuge der Durchführung der Mammographie eine falsche Beurteilung erfolgt oder gar ein Mammakarzinom nicht identifiziert wird, folglich eine fehlerhafte Diagnose durchgeführt wird.

Überraschenderweise konnten die Erfinder feststellen, dass durch die Applikation von Dopamin-Antagonisten die Brustdichte verringert wird und infolgedessen eine sichere Diagnose eines Mammakarzinoms in der Durchführung einer Mammographie erfolgen kann.

Besonders vorteilhaft kann durch eine Verringerung der Brustdichte mittels Dopamin-Agonisten eine sichere Diagnose eines Mammakarzinoms mittels einer Mammographie erfolgen. Weiterhin ist bevorzugt das durch die Vergabe bzw. Applikation eines Dopamin-Agonisten die Brustdichte um mindestens eine Stufe reduziert wird.

Daher betrifft Erfindung ein entsprechendes Verfahren oder ein Mittel unter Verwendung eines Dopamin-Agonisten zur Diagnose und / oder Risikostratifizierung eines Mammakarzinoms mithilfe einer Mammographie gemäß den vorliegenden Patentansprüchen.

Daher betrifft die Erfindung ein Verfahren zur Diagnose und / oder Risikostratifizierung von Brustkrebs, wobei mittels eines Dopamin-Agonisten eine Mammographie erfolgt.

Insbesondere betrifft die Erfindung ein Verfahren zur Diagnose und / oder Risikostratifizierung von Brustkrebs, wobei der Dopamin-Agonist einem Patienten zur Verringerung der Brustdichte appliziert wird.

Weiterhin betrifft die Erfindung ein Mittel enthaltend einen Dopamin-Agonisten zur Verwendung in einem Verfahren zur Diagnose und / oder Risikostratifizierung von Brustkrebs, wobei eine Mammographie erfolgt.

Insbesondere betrifft die Erfindung ein Mittel enthaltend einen Dopamin-Agonisten zur Verwendung in einem Verfahren zur Diagnose und / oder Risikostratifizierung von Brustkrebs, wobei der Dopamin-Agonist einem Patienten zur Verringerung der Brustdichte appliziert wird.

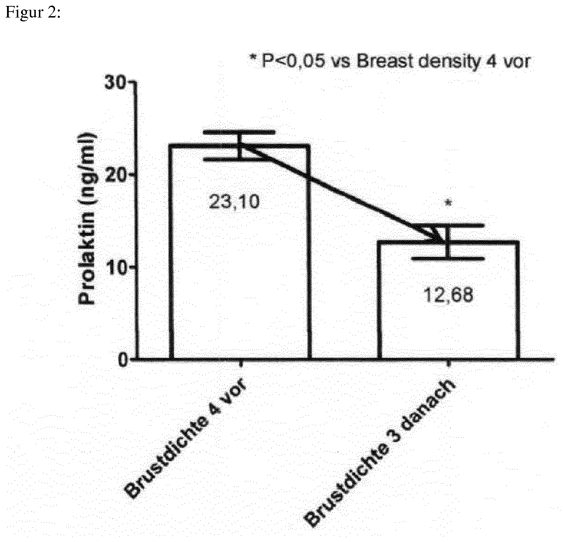

Der Dopamin-Agonist wird vorzugsweise in einem längeren Zeitraum dem Patienten appliziert, vorzugsweise oral appliziert. Beispielsweise können die Erfinder zeigen, dass bei einer einjährigen Applikation von einem Pflanzenextrakt aus Vitex Agnus Castus die Brustdichte um eine Stufe verringert werden kann, wie in Figur 2 dargelegt.

In einer besonders bevorzugten Ausführungsform der Erfindung ist der Dopamin-Agonist ein Pflanzenextrakt aus Vitex Agnus Castus.

Geeignete Dopamin-Agonisten sind erfindungsgemäß weiterhin Bromocriptin, Cabergolin, Apomorphin, Pramipexol, Quinagolid, Ropinirol oder Rotigotin.

Im Rahmen dieser Erfindung ist unter einem Pflanzenextrakt aus Vitex Agnus Castus ein durch wässrige und/oder ethanolische Extraktion oder durch Extraktion mit Kohlendioxid, vorzugweise der Früchte und/oder Blätter von Vitex Agnus Castus erhältlicher Pflanzenextrakt zu verstehen. Bevorzugte Herstellungsverfahren sind bspw. in EP 0 753 306 B1 und EP 1 368 605 B1 beschrieben. Ein erfindungsgemäß geeigneter Pflanzenextrakt ist das hergestellte Arzneimittel Agnucaston® der Anmelderin.

Im Rahmen dieser Erfindung versteht man unter Brustkrebs (Synonym: Mammakarzinom) ein Karzinom der Brustdrüse (Mamma).

Ein Mammakarzinom ist die mit Abstand häufigste Krebserkrankung bei Frauen. Häufigstes Frühsymptom ist eine palpable indolente Resistenz in der Brust. Die Behandlung eines solchen Mammakarzinoms erfolgt operativ und/oder systemisch über eine Hormontherapie, Chemotherapie und Strahlentherapie.

Die Diagnose eines Brustkrebses erfolgt mithilfe der Mammographie und bedarf zum Beispiel der optischen oder optisch-gestützten Auswertung.

Im Rahmen dieser Erfindung wird unter Mammographie eine Nativ-Röntgenaufnahme der Brust in verschiedenen Projektionen (insbesondere kraniokaudal, mediolateral) verstanden, die ggfs. mit einer zusätzlichen Spezialaufnahme der Achsel (Axilla) verbunden ist.
Ferner ist es üblich, eine sogenannte Rastertechnik anzuwenden.

Bei der Beurteilung und Identifizierung eines Mammakarzinoms ist ein unscharf begrenzter Rundherd mit seitendifferenten suspekten Verdichtungen mit radiären Ausläufern und gruppierten Polymorphe-Mikroverkalkungen heranzuziehen.
Die Mammographie wird zur Diagnostik eines Mammakarzinoms als auch deren Präkanzerosen eingesetzt.

Die Mammographie ermöglicht daher die Objektivierung und Lokalisation eines pathologischen Testbefunds, z. B. bei Mastopathie, sezernierende Mamille und Thelorrhagie.

Ferner wird die Mammographie zur Überwachung von Risikopatienten bzw. das Screening von gesunden Kollektiven eingesetzt, insbesondere im Rahmen der Krebsfrüherkennungsuntersuchungen (Frauen ab 50 Jahre) als auch in der Nachversorgung (Monitoring).

Besonders vorteilhaft kann das erfindungsgemäße Verfahren oder die eingesetzten Mittel die Qualität der Diagnose einer Mammographie erhöhen und folglich die diagnostische Wertigkeit als auch die Risikostratifizierung der Patienten verbessern.

Daher betrifft die Erfindung ebenfalls ein Verfahren oder ein erfindungsgemäßes Mittel enthaltend einen Dopamin-Agonisten zur Verwendung in einem Verfahren zur Diagnose und / oder Risikostratifizierung von Patienten eines Mammakarzinoms zur Durchführung von klinischen Entscheidungen, wie weiterführende Behandlung und Therapie mittels Arzneimitteln, vorzugsweise in der zeitlich kritischen Intensivmedizin, einschließlich der Entscheidung zur Hospitalisierung des Patienten.

In einer weiteren bevorzugten Ausführungsform betrifft daher das erfindungsgemäße Verfahren oder ein erfindungsgemäßes Mittel enthaltend einen Dopamin-Agonisten zur Verwendung in einem Verfahren zur Therapiesteuerung von Brustkrebs.

In einer weiteren bevorzugten Ausführungsform des erfindungsgemäßen Verfahrens oder ein erfindungsgemäßes Mittel enthaltend einen Dopamin-Agonisten zur Verwendung in einem Verfahren, erfolgt die Diagnose und / oder Risikostratifizierung zur Prognose, zur differentialdiagnostischen Früherkennung und Erkennung, zur Beurteilung des Schweregrades und zur therapiebegleitenden Verlaufsbeurteilung.

Der Begriff "Risikostratifizierung" umfasst erfindungsgemäß das Auffinden von Patienten, insbesondere Risikopatienten, mit der schlechteren Prognose, zwecks intensiverer Diagnostik und Therapie/Behandlung von Mammakarzinom mit dem Ziel einen möglichst günstigen Verlauf der Krankheit zu ermöglichen.

Aufgrund der hervorragenden Eigenschaft des erfindungsgemäßen Pflanzenextraktes aus Vitex Agnus Castus zur Verringerung der Brustdichte, ist ein solcher Pflanzenextrakt aus Vitex Agnus Castus zur Prophylaxe und Behandlung von Brustkrebs besonders geeignet. Daher betrifft die Erfindung ebenfalls ein Arzneimittel enthaltend ein Pflanzenextrakt aus Vitex Agnus Castus zur Prophylaxe und Behandlung von Brustkrebs.

Das erfindungsgemäße Arzneimittel kann einem Patienten appliziert werden, insbesondere oral appliziert werden.

Die erfindungsgemäßen Mittel oder das erfindungsgemäße Arzneimittel kann in einer Zubereitung in Form von Dosierungseinheiten hergestellt werden. Dies bedeutet, dass die Zubereitungen in Form einzelner Teile, vorzugsweise Kapseln und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge des erfindungsgemäßen (Arznei-)Mittels, die bei einer Applikation verabreicht wird und die gewöhnlich einer ganzen, einer halben, einem Drittel oder einem Viertel einer Tagesdosis entspricht. Bevorzugt ist die Arzneiform einer Tablette mit geeigneten Trägerstoffen. Solche nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen, wie a) Füll- und Streckmittel, z.B. Stärken, Milchzucker, Rohrzucker, Glukose, Mannit, Dextrine, Maltodextrin und Kieselsäure, hochdisperses Siliciumdioxid, b) Bindemittel, z.B. Carboxymethylcellulose, Cellulosepulver, mikrokristalline Cellulose, Alginate, Gelatine, Polyvinylpyrrolidon, c) Feuchthaltemittel, z.B. Glycerin, d) Sprengmittel, z.B. Agar-Agar, Calciumcarbonat und Natriumcarbonat, e) Lösungsverzögerer, z.B. Paraffin und f) Resorptionsbeschleuniger, z.B. quartäre Ammoniumverbindungen, g) Netzmittel, z.B. Cetylalkohol, Glycerinmonostearat, h) Adsorptionsmittel, z.B. Kaolin und Bentonit und i) Gleitmittel, z.B. Talkum, Calcium- und Magnesiumstearat und feste Polyethylenglykole oder Gemische der unter a) bis i) aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein, z.B. solche wie nicht abschließend Hypromellose, mikrokristalline Cellulose, Stearinsäure, Titandioxid, und ebenfalls so zusammengesetzt sein, dass sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Die Figur 1a zeigt die Korrelation des Prolaktin-Spiegels zur Brustdichte unter Berücksichtigung der Stufen 1-4 bei Patienten (n = 50).

Die Figur 1b zeigt für Mastodynie Patienten (n=21) die Korrelation des Prolaktin-Spiegels zur Brustdichte unter Berücksichtigung der Stufen 1-4. Ein Patient ist berücksichtigt nach einseitiger Mastektomie.

Die Figur 2 zeigt den Prolaktin-Spiegel bei Frauen mit Brustdichte 4 vor und nach einjähriger Einnahme von einem Pflanzenextrakt aus Vitex Agnus Castus.

## Patentansprüche

1. Verfahren zur Diagnose und / oder Risikostratifizierung von Brustkrebs, **dadurch gekennzeichnet, dass** mittels eines Dopamin-Agonisten eine Mammographie erfolgt.

2. Verfahren zur Diagnose und / oder Risikostratifizierung von Brustkrebs nach Anspruch 1, **dadurch gekennzeichnet, dass** der Dopamin-Agonist einem Patienten zur Verringerung der Brustdichte appliziert wird.

3. Verfahren zur Diagnose und / oder Risikostratifizierung von Brustkrebs nach Anspruch 1 oder Anspruch 2, **dadurch gekennzeichnet, dass** der Dopamin-Agonist ein Pflanzenextrakt aus Vitex Agnus Castus ist.

4. Verfahren zur Diagnose und / oder Risikostratifizierung von Brustkrebs nach Anspruch 1 oder Anspruch 2, **dadurch gekennzeichnet, dass** der Dopamin-Agonist ausgewählt ist aus der Gruppe Bromocriptin, Cabergolin, Apomorphin, Pramipexol, Quinagolid, Ropinirol oder Rotigotin.

5. Mittel enthaltend einen Dopamin-Agonisten zur Verwendung in einem Verfahren zur Diagnose und / oder Risikostratifizierung von Brustkrebs, wobei eine Mammographie erfolgt.

6. Mittel enthaltend einen Dopamin-Agonisten zur Verwendung in einem Verfahren zur Diagnose und / oder Risikostratifizierung von Brustkrebs nach Anspruch 5, **dadurch gekennzeichnet, dass** der Dopamin-Agonist einem Patienten zur Verringerung der Brustdichte appliziert wird.

7. Mittel enthaltend einen Dopamin-Agonisten zur Verwendung in einem Verfahren zur Diagnose und / oder Risikostratifizierung von Brustkrebs nach Anspruch 5 oder Anspruch 6, dass der Dopamin-Agonist ein Pflanzenextrakt aus Vitex Agnus Castus ist.

8. Mittel enthaltend einen Dopamin-Agonisten zur Verwendung in einem Verfahren zur Diagnose und / oder Risikostratifizierung von Brustkrebs nach Anspruch 5 oder Anspruch 6, dass der Dopamin-Agonist ausgewählt ist aus der Gruppe Bromocriptin, Cabergolin, Apomorphin, Pramipexol, Quinagolid, Ropinirol oder Rotigotin.

9. Mittel enthaltend einen Dopamin-Agonisten zur Verwendung in einem Verfahren zur Diagnose und / oder Risikostratifizierung von Brustkrebs nach einem der Ansprüche 5-8 zur Durchführung von klinischen Entscheidungen, insbesondere weiterführende Behandlungen und Therapien mittels Arzneimitteln, insbesondere in der Intensivmedizin, einschließlich der Entscheidung zur Hospitalisierung des Patienten.

10. Mittel enthaltend einen Dopamin-Agonisten zur Verwendung in einem Verfahren zur Diagnose und / oder Risikostratifizierung von Brustkrebs nach einem der Ansprüche 5-8 zur Prognose, zur differentialdiagnostischen Früherkennung und Erkennung, zur Beurteilung des Schweregrades und zur therapiebegleitenden Verlaufsbeurteilung.

11. Arzneimittel enthaltend ein Pflanzenextrakt aus Vitex Agnus Castus zur Prophylaxe und Behandlung von Brustkrebs.
